# EUROPEAN PATENT APPLICATION

(11) **EP 3 295 817 A2**
(43) Date of publication of application: **21.03.2018**
(21) Application number: 16807842.6
(22) Date of filing: 10.06.2016
(51) Int. Cl.: A44C 5/00

(54) **APPARATUS AND METHOD FOR ASSISTING CHEST COMPRESSIONS**

(30) Priority: 12.06.2015 KR 20150083342; 03.06.2016 KR 20160069446
(71) Applicant: Credo Co., Ltd, Wonju-si, Gangwon-do 26493 (KR)
(72) Inventor: CHOI, Jong Kuk, Goyang-si Gyeonggi-do 10483 (KR); HAM, Seung Mun, Wonju-si Gangwon-do 26494 (KR); WANG, Ji Hyo, Wonju-si Gangwon-do 26435 (KR); LEE, Dong Joon, Goyang-si Gyeonggi-do 10416 (KR)
(74) Representative: Manitz Finsterwald Patentanwälte PartmbB
(86) International application number: PCT/KR2016/006179
(87) International publication number: WO 2016/200202

(57) **Abstract**

An apparatus for assisting chest compressions includes a main unit that generates information relevant to chest compressions, and a connecting part which is extended from the main unit and of which both ends are connected to each other in order for the apparatus to be wearable on a part of a user's body, and the main unit includes a sensor unit configured to measure a compression depth during the chest compressions and measure a compression direction during the chest compressions, an information generating unit configured to generate information on the basis of a result of the measurement, and a light source operating unit configured to operate a light source on the basis of the information.

## Description

### TECHNICAL FIELD

The present disclosure relates to an apparatus and method for assisting chest compressions and relates to an apparatus and method for assisting a subject requiring CPR to get CPR.

### BACKGROUND

CPR (Cardiopulmonary resuscitation) is an emergency procedure to restore normal breathing and circulation in a person who is in an accident such as heart attack or drowning and includes cleaning the airway connected to the lung and performing chest compressions for applying external pressure to the chest, and must be performed only to those who are unconscious with no breathing by a CPR-trained person.

In the CPR, chest compression is a method for assisting blood circulation in a patient. In the patient who is in cardiac arrest, blood flow to vital organs such as brain and heart is stooped, and, thus, blood circulation can be maintained to a certain degree by compressing the chest of the patient.

Referring to the Standardized Official 2010 Guidelines for CPR, it is recommended to perform chest compressions at a compression rate of at least 100 per minute with a compression depth of at least 2 inches, but in practice, a compression rate or a compression depth has often not reached the recommended level. Further, it is recommended to assist patients with an endotracheal tube in ventilating their lungs at a rate of 8 to 10 per minute, but in many cases, hyperventilation occurs. Hyperventilation causes an increase in intrapleural pressure and thus lowers coronary perfusion pressure, resulting in a low probability of spontaneous circulation recovery.

Meanwhile, bracelets are accessories used to add the beauty and easy to wear and thus easy to carry. Therefore, many people are wearing bracelets. Accordingly, there has been a growing need for an auxiliary device which is easy to wear and enables CPR to be accurately performed.

The background technology of the present disclosure is disclosed in Koran Patent No. 10-1355963.

### DISCLOSURE OF THE INVENTION

### PROBLEMS TO BE SOLVED BY THE INVENTION

In view of the foregoing, the present disclosure provides an apparatus for assisting chest compressions which is wearable on a part of a user's body. Further, the apparatus is provided to increase the accuracy of chest compressions by guiding a CPR performer about an accurate chest compression depth and an accurate chest compression rate by sound.

Further, the apparatus is provided to advance the time of arrival of rescue workers by connection with a smart device of the CPR performer during CPR. Furthermore, the apparatus is provided to improve objectivity by digitizing and recording all the processes of CPR from start to end and increase the efficiency and transparency of emergency treatment. Also, the apparatus is provided to enable the CPR performer to easily check the apparatus.

However, problems to be solved by the present disclosure are not limited to the above-described problems. There may be other problems to be solved by the present disclosure.

### MEANS FOR SOLVING THE PROBLEMS

As a technical means for solving the above-described technical problems, an apparatus for assisting chest compressions according to an exemplary embodiment of the present disclosure includes a main unit that generates information relevant to the chest compressions, and a connecting part which is extended from the main unit and of which both ends are connected to each other in order for the apparatus to be wearable on a part of a user's body, and the main unit includes a sensor unit configured to measure a compression depth during the chest compressions and measure a compression direction during the chest compressions, an information generating unit configured to generate information on the basis of a result of the measurement, and a light source operating unit configured to operate a light source on the basis of the information.

Further, according to an example of the present exemplary embodiment, the light source operating unit may operate multiple light sources having different colors, and if the compression depth is within a first threshold range, the light source operating unit may operate a first color light source, and if the compression depth is within a second threshold range, the light source operating unit may operate both the first color light source and a second color light source.

Furthermore, according to an example of the present exemplary embodiment, the light source operating unit may turn on some of the multiple light sources depending on the compression depth, and the number of some light sources may vary depending on the compression depth.

Moreover, according to an example of the present exemplary embodiment, the light source operating unit may differentiate the number or color of light sources to be operated with a first compression depth in a first compression direction from the number or color of light sources to be operated with the first compression depth in a second compression direction.

Further, according to an example of the present exemplary embodiment, the first threshold range and the second threshold range may be determined on the basis of at least one of age or gender of a subject of the chest compressions.

Furthermore, according to an example of the present exemplary embodiment, the sensor unit may measure at least one of the compression depth and the compression direction using an acceleration sensor.

Moreover, according to an example of the present exemplary embodiment, the apparatus for assisting chest compressions may further include a communication unit that transmits the information to an external device.

Further, according to an example of the present exemplary embodiment, the external device may be another apparatus for assisting chest compressions.

Furthermore, according to an example of the present exemplary embodiment, the other apparatus for assisting chest compressions may be worn on a part of a second user's body, and the other apparatus for assisting chest compressions may operate using the information

Moreover, according to an example of the present exemplary embodiment, the other apparatus for assisting chest compressions may be worn on another part of the user's body, and the communication unit may receive external information from the other apparatus for assisting chest compressions and the information generating unit may generate the information considering the external information.

Further, according to an example of the present exemplary embodiment, the external device may be a mobile device which is communication-connected with at least two apparatuses for assisting chest compressions.

Furthermore, according to an example of the present exemplary embodiment, the information may be accuracy information indicating whether or not the chest compressions are accurately performed.

Moreover, according to an example of the present exemplary embodiment, the sensor unit may measure at least one of the compression depth and the compression angle on the basis of an offset and a signal change cycle depending on a change in acceleration of the main unit.

Further, according to an example of the present exemplary embodiment, a detachable part may be formed between the main unit and the connecting part and may fix the main unit to be detached or attached.

Furthermore, according to an example of the present exemplary embodiment, the communication unit may transmit a current position of the user to the external device while the user performs the chest compressions.

Moreover, according to an example of the present exemplary embodiment, the main unit may further include a switch configured to receive a user input to start providing the information relevant to the chest compressions and a sound output unit configured to output sound to guide the user about the chest compressions while the user performs the chest compressions.

Further, according to an example of the present exemplary embodiment, if an operation of a predetermined type is input through the switch, the first threshold range and the second threshold range may be changed on the basis of at least one of age or gender of a subject of the chest compressions.

Furthermore, according to an example of the present exemplary embodiment, the sensor unit may be a 6-axis acceleration sensor, and the sensor unit may measure a movement distance from the chest in a direction toward a reference axis as the compression depth.

A method for assisting chest compressions using an apparatus according to an exemplary embodiment of the present disclosure includes generating information relevant to the chest compressions by a main unit and making a user wear the apparatus on a part of his/her body by extending a connecting part from the main unit and connecting both ends of the connecting part to each other, and the generating of information relevant to the chest compressions includes measuring a compression depth during the chest compressions and a compression direction during the chest compressions, generating the information on the basis of a result of the measurement, and operating a light source on the basis of the information.

The above-described means for solving the problems are provided by way of illustration only and should not be construed as liming the present disclosure. Besides the above-described exemplary embodiments, there may be additional exemplary embodiments described in the accompanying drawings and the detailed description.

### EFFECTS OF THE INVENTION

According to the above-described means for solving the problems, the present disclosure can provide an apparatus for assisting chest compressions which is wearable on a part of a user's body. Further, the apparatus can increase the accuracy of chest compressions by guiding a CPR performer about an accurate chest compression depth and an accurate chest compression rate by sound.

Further, the apparatus can make it easy to ask for help and advance the time of arrival of rescue workers by connection with a smart device of the CPR performer during CPR. Furthermore, the apparatus can improve objectivity by digitizing and recording all the processes of CPR from start to end and increase the efficiency and transparency of emergency treatment.

The apparatus can increase the accuracy of basic CPR to be performed by emergency medical workers in case of acute heart attack and thus increase the survival rate of patients, and automatically record emergency medical records which the emergency medical workers have to record and thus suppress confusion in a medical record about an emergency, and can also objectively record the processes of CPR. Further, these records can be a legal safeguard for the CPR performer.

### BRIEF DESCRIPTION OF THE DRAWINGS

**FIG. 1** is a configuration view of a system for assisting chest compressions according to an exemplary embodiment of the present disclosure;
**FIG. 2** is a configuration view of a main unit according to an exemplary embodiment of the present disclosure;
**FIG. 3** is a diagram illustrating an operation of an apparatus for assisting chest compressions according to an exemplary embodiment of the present disclosure;
**FIG. 4A** to **FIG. 4B** are diagrams illustrating an operation of a sensor unit according to an exemplary embodiment of the present disclosure;
**FIG. 5** is a diagram illustrating an operation of a light source operating unit according to an exemplary embodiment of the present disclosure;
**FIG. 6** is a flowchart illustrating a process of chest compressions according to an exemplary embodiment of the present disclosure; and
**FIG. 7** is a flowchart illustrating a method for assisting chest compressions using an apparatus according to an exemplary embodiment of the present disclosure.

### BEST MODE FOR CARRYING OUT THE INVENTION

Hereinafter, embodiments of the present disclosure will be described in detail with reference to the accompanying drawings so that the present disclosure may be readily implemented by those skilled in the art. However, it is to be noted that the present disclosure is not limited to the embodiments but can be embodied in various other ways. In drawings, parts irrelevant to the description are omitted for the simplicity of explanation, and like reference numerals denote like parts through the whole document.

Through the whole document, the term "connected to" or "coupled to" that is used to designate a connection or coupling of one element to another element includes both a case that an element is "directly connected or coupled to" another element and a case that an element is "electronically connected or coupled to" another element via still another element.

Through the whole document, the terms "on", "above", "on an upper end", "below", "under", and "on a lower end" that are used to designate a position of one element with respect to another element include both a case that the one element is adjacent to the other element and a case that any other element exists between these two elements.

Further, through the whole document, the term "comprises or includes" and/or "comprising or including" used in the document means that one or more other components, steps, operation and/or existence or addition of elements are not excluded in addition to the described components, steps, operation and/or elements unless context dictates otherwise.

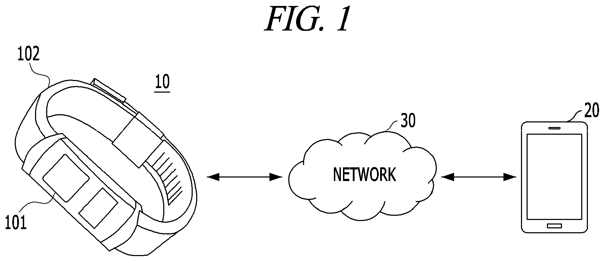

**FIG. 1** is a configuration view of a system for assisting chest compressions according to an exemplary embodiment of the present disclosure. Referring to **FIG. 1**, the system for assisting chest compressions includes a chest compression assisting apparatus 10 for assisting chest compressions and an external device 20 connected to the chest compression assisting apparatus 10 through a network 30. However, the system for assisting chest compressions is not limited to the above-described configuration example. For example, the chest compression assisting apparatus 10 may be connected to another chest compression assisting apparatus 10 through the network or multiple chest compression assisting apparatuses 10 may be connected to the external device 20 through the network.

The external device 20 according to various exemplary embodiments of the present disclosure may include, for example, at least one of a smartphone, a tablet personal computer (PC), a mobile phone, a video phone, an e-book reader, a desktop PC, a laptop PC, a netbook computer, a workstation, a server, a personal digital assistant (PDA), a portable multimedia player (PMP), a MP3 player, a mobile medical device, a camera, a wearable device (e.g., smart glasses, a head mounted-device (HMD)), an electronic garment, an electronic bracelet, an electronic necklace, an electronic appcessory, an electronic tattoo, a smart mirror, or a smart watch.

The external device 20 may receive information generated on the basis of a measurement result of the chest compression assisting apparatus 10, and the external device 20 may analyze information on the basis of the received information or ask for help.

The chest compression assisting apparatus 10 and the external device 20 may be connected to each other through the network 30. The network 30 refers to a connection structure that enables information exchange between nodes such as devices, servers, etc. and may include wireless communication and wired communication. The wireless communication may use, for example, at least one of long term evolution (LTE), LTE-advanced (LTE-A), code division multiple access (CDMA), wideband CDMA (WCDMA), universal mobile telecommunications system (UMTS), wireless broadband (WiBro), or global system for mobile communications (GSM), and the like, as a cellular communication protocol. Further, the wireless communication may include, for example, local area communication. The local area communication may include, for example, at least one of Wi-Fi, Bluetooth, near field communication (NFC), global positioning system (GPS), or the like. The wired communication may include, for example, at least one of universal serial bus (USB), high definition multimedia interface (HDMI), recommended standard 232 (RS-232), plain old telephone service (POTS), or the like. The network 30 may include at least one of telecommunications networks such as a computer network (e.g., LAN or WAN), Internet, or telephone network.

The chest compression assisting apparatus 10 may provide visual and audio information relevant to chest compressions including CPR to a user who is performing the chest compressions, and the chest compression assisting apparatus 10 may include a main unit 101 and a connecting part 102. In the present disclosure, CPR refers to chest compressions for compressing the chest during cardiopulmonary resuscitation unless specifically indicated, and the use of the terms CPR and chest compressions together cannot change their meanings. Further, the terms CPR and chest compressions can be used together unless specifically indicated.

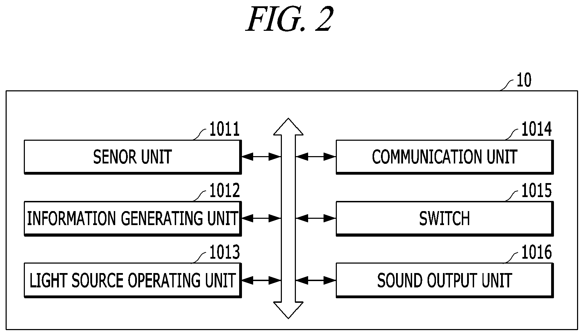

The main unit 101 may generate information relevant to chest compressions, and the main unit 101 will be described in detail with reference to **FIG. 2****.**

The connecting part 102 may be extended from the main unit 101 and both ends thereof are connected to each other, and, thus, the user can wear the chest compression assisting apparatus 10 on a part of his/her body. In an example according to various exemplary embodiments of the present disclosure, the connecting part 102 may enable the chest compression assisting apparatus 10 to be fixed to a wrist of the user. More specifically, the connecting part 102 may be formed of urethane, but is not limited thereto, and the connecting part 102 may include a groove to be engaged with a rotating part to be described later and thus enable the main unit 101 to be rotated by the rotating part. Further, the connecting part 102 may be joined in various ways in order for the chest compression assisting apparatus 10 to be easily worn on the hand or wrist. Specifically, the connecting part 102 may be joined by a cable tie type fastening using Velcro, snap buttons, magnets, or grooves and protrusions. Therefore, it is possible for the user to easily carry the chest compression assisting apparatus 10.

The rotating part may be formed between the main unit 101 and the connecting part 102 and may rotate the main unit 101 according to a movement of the user. For example, the rotating part may be a rotatory mechanical structure which can be rotated by 45 degrees or more, and when the user performs an operation of compressing the chest, the rotating part rotates the main unit 101 according to a movement of the user's flexed wrist and thus enables the user to look at the main unit 101. The rotating part is designed to solve the problem that when the user performs chest compressions, the user' wrist is flexed, and, thus, the user cannot check a light source in the main unit 101 well. Therefore, it is possible for the user to more easily check the light source.

According to another exemplary embodiment of the present disclosure, the chest compression assisting apparatus 10 may include a detachable part (not illustrated). The detachable part may be formed between the main unit 101 and the connecting part 102 and may fix the main unit 101 to be detached from or attached to the chest compression assisting apparatus 10. In other words, the detachable part may separate or connect the chest compression assisting apparatus 10 and the main unit 101. The detachable part may separate the chest compression assisting apparatus 10 and the main unit 101 and enable the main unit 101 to operate and a main unit 101 of another chest compression assisting apparatus 10 to be attached and fixed to chest compression assisting apparatus 10.

The detachable part may provide the convenience in use including transporting, charging, or storing the main unit 10, and may also provide the expandability to use the main unit 101 of the other chest compression assisting apparatus 10.

Although not illustrated in the drawings, a power supply unit (not illustrated) and a charging unit (not illustrated) may be further included in the chest compression assisting apparatus 10. The power supply unit may supply power to the chest compression assisting apparatus 10. For example, the power supply unit may be provided as a lithium battery within the chest compression assisting apparatus 10, but is not limited thereto.

The charging unit may be provided on one side of the main unit 101 to charge the chest compression assisting apparatus 10. For example, the charging unit may be provided to charge the power supply unit and configured as a Micro 5 pin. However, the charging unit is not limited to the above-described configuration and can be applied with various configurations, for example, using wireless charging. Meanwhile, the charging unit may be used as an interface through which information generated in the main unit 101 can be transmitted to the external device 20 over wires.

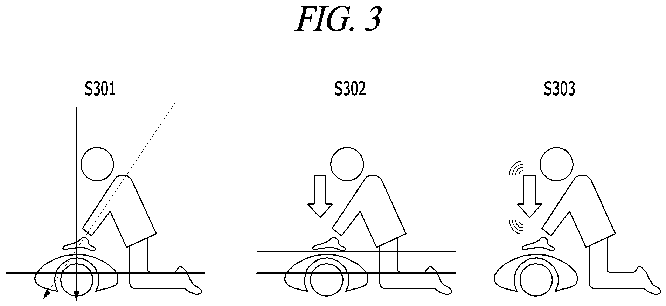

**FIG. 3** is a diagram illustrating an operation of an apparatus for assisting chest compressions according to an exemplary embodiment of the present disclosure. Referring to FIG. 3, in a process S301, when a performer performs chest compressions, the chest compression assisting apparatus 10 may estimate an angle of the performer's arm to more accurately perform chest compressions, and in a process S302, the chest compression assisting apparatus 10 may measure a compression depth when the performer compresses the chest. Through the process S301 to the process S302, the chest compression assisting apparatus 10 can determine whether or not the performer compresses the chest at an accurate angle and thus improve the accuracy of the chest compressions including CPR. In a process S303, the chest compression assisting apparatus 10 may output sound to guide the performer about CPR and thus enable the performer to more accurately perform CPR.

**FIG. 2** is a configuration view of a main unit according to an exemplary embodiment of the present disclosure. Referring to **FIG. 2**, the main unit 101 may include a sensor unit 1011, an information generating unit 1012, a light source operating unit 1013, a communication unit 1014, a switch 1015, and a sound output unit 1016. However, the main unit 101 is not limited to the above-described configuration.

The sensor unit 1011 may measure a compression depth and a compression direction during chest compressions. The sensor unit 1011 may measure at least one of the compression depth and the compression direction using an acceleration sensor, and may measure at least one of the compression depth and a compression angle on the basis of an offset and a signal change cycle depending on a change in acceleration of the main unit. For example, the sensor unit 1011 may be a 6-axis acceleration sensor and may measure a movement distance from the chest in a direction toward a reference axis as the compression depth.

More specifically, the sensor unit 1011 may detect a change in position of the main unit 101, and in this case, the sensor unit 1011 may be an acceleration sensor. The acceleration sensor refers to a sensor configured to measure dynamic force, such as vibration acceleration, shock, etc., of an object and detect a motional state of the object. In other words, when chest compressions are performed, the sensor unit 1011 may measure a movement with a distance of 4 cm to 6 cm from the chest toward the heart of a subject of the chest compressions.

The information generating unit 1012 may generate information on the basis of a measurement result of the sensor unit 1011. The information may be accuracy information indicating whether or not the chest compressions are accurately performed. For example, when the sensor unit 1011 measures a movement with a distance of 4 cm to 6 cm from the chest toward the heart of the subject of the chest compressions, the information generating unit 1012 may generate information indicating that the chest is accurately compressed.

The sensor unit 1101 may continuously sense a motion of the user, and if the sensor unit 1101 measures an acceleration pattern of CPR a predetermined number of times or more, the information generating unit 1012 may determine that CPR is being performed and may also enable the chest compression assisting apparatus 10 to operate. Accordingly, in the case where CPR or chest compressions are needed using the chest compression assisting apparatus 10, there may be no time to operate the chest compression assisting apparatus 10. In this case, even if the user does not operate the chest compression assisting apparatus 10, the chest compression assisting apparatus 10 is automatically powered on and provides a guide about chest compressions. Therefore, it is possible to increase the convenience of the user.

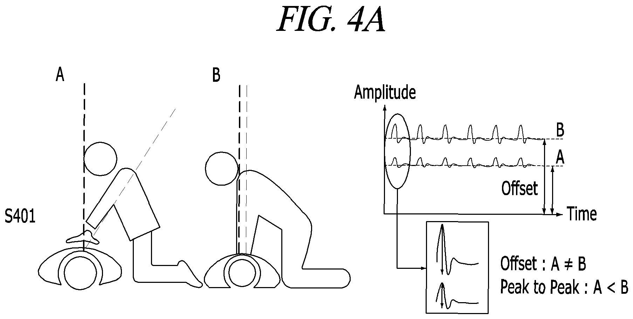

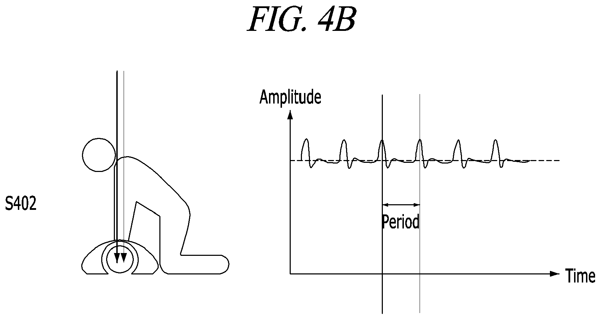

Hereinafter, operations of the sensor unit 1011 and the information generating unit 1012 will be described in detail with reference to **FIG. 4A** to **FIG. 4B. FIG. 4A** to **FIG. 4B** are diagrams illustrating an operation of a sensor unit according to an exemplary embodiment of the present disclosure.

Referring to **FIG. 4A** to **FIG. 4B**, the sensor unit 1011 may be designed as a 6-axis accelerometer driving circuit for measuring a movement of the wrist of the user who is performing chest compressions including CPR, and may measure parameters calculated while CPR or chest compressions are performed.

Referring to a process S401, the sensor unit 1011 may estimate an angle of the arm when CPR or chest compressions are performed. There is a difference in height of offset between the case where chest compressions are performed at a non-vertical angle of the arm with respect to the chest (A) and the case where chest compressions are performed at a vertical angle of the arm with respect to the chest (B), and the height of the maximum offset of a reference axis (a direction toward the heart from the chest of the subject of the chest compressions) may be measured higher in the case B than the case A. In this case, the height of the maximum offset refers to a movement distance of the main unit 101 toward the reference axis during the chest compressions, and, thus, the sensor unit 1011 may estimate an angle of the arm of the chest compression performer. According to another exemplary embodiment, in the case A, the sensor unit 1011 may measure an offset value of another axis instead of the reference axis and may also estimate an angle of the arm during chest compressions on the basis of an offset variation between different axes.

The information generating unit 1012 may generate accuracy information indicating whether or not chest compressions are accurately performed on the basis of the measured height of the maximum offset or the estimated angle of the arm.

Referring to a process S402, the sensor unit 1011 may estimate the number and cycle of chest compressions using measurement values and signal patterns measured while CPR or chest compressions are performed. More specifically, the sensor unit 1011 can measure the number of chest compressions can be measured on the basis of a 6-axis acceleration signal pattern or measurement cycle. Meanwhile, the information generating unit 1012 may generate information indicating whether or not chest compressions are performed with an accurate cycle on the basis of the measured number of chest compressions.

The information generating unit 1012 may generate information considering a rotation angle of the rotating part. For example, when the main unit 101 is rotated by the rotating part, the information generating unit 1012 may correct a measurement result of the sensor unit 1011 with reference to a rotation angle of the main unit 101 as a reference angle.

The light source operating unit 1013 may operate a light source on the basis of the generated information. In the main unit 101, multiple light sources having different colors are positioned, and the light source operating unit 1013 may operate the multiple light sources having different colors. If a compression depth is within a first threshold range, the light source operating unit 1013 may operate a first color light source, and if the compression depth is within a second threshold range, the light source operating unit 1013 may operate both the first color light source and a second color light source. Further, the light source operating unit 1013 may turn on some of the multiple light sources depending on the compression depth, and in this case, the number of some light sources may vary depending on the compression depth. In this case, the first threshold range and the second threshold range may be determined on the basis of at least one of age or gender of the subject of the chest compressions.

For example, the light source operating unit 1013 may estimate a depth of chest compressions to quantify parameters relevant to CPR. In this case, the light source operating unit may estimate a depth of chest compressions performed by the performer by double integration of at least one of the compression depth, compression direction and compression rate measured by the sensor unit 1011 or regression equation modeling according to a relationship between the compression rate and the compression depth, and then operate the multiple light sources on the basis of the estimated depth of chest compressions. The performer may estimate a compression depth from the operated light sources and determine whether or not he/she has compressed the chest to an accurate depth.

According to another exemplary embodiment, the light source operating unit 1013 is provided on both sides of the main unit 101 and thus enables the chest compression performer to check the operated light sources and thus more accurately perform chest compressions. In this case, the operated light sources may have at least colors and may include multiple light sources. More specifically, if the information generating unit 1012 determines that the chest compressions have not been accurately performed on the basis of the measurement result of the sensor unit 1011, a light source may be turned on in red, and if the chest compressions are accurately performed, multiple light sources including the previously turned on light source may be turned on in green. In other words, if the chest compressions are accurately performed, multiple red, yellow, and green light sources may be stacked and turned on.

According to another exemplary embodiment, the light source operating unit 1013 may turn on the multiple light sources on the basis of the measurement result of the sensor unit 1011. For example, if the information generating unit 1012 determines that a compression depth of chest compressions exceeds a predetermined threshold value and the chest compressions have not been accurately performed on the basis of the measurement result of the sensor unit 1011, the light source operating unit 1013 may turn on the light sources in red. In this case, a red light source which is turned on may be stacked on a green light source and the red light source stacked on the green light source may be different from the above-described red light source. In other words, if a depth of chest compressions exceeds a predetermined threshold value, at least one of red, yellow, and green lights and a red light may be stacked and turned on.

According to another exemplary embodiment, the light source operating unit 1013 may operate a light source on the basis of other information in addition to depth information of chest compressions. For example, if a battery power level of the chest compression assisting apparatus 10 is 30% or less, the light source operating unit 1013 may display in yellow a warning signal indicating that the chest compression assisting apparatus 10 needs to be charged. In this case, the yellow light source may be turned on for 2 minutes at hourly intervals, but is not limited thereto. Accordingly, information about the chest compressions can be simply and accurately transferred to the chest compression performer, and, thus, CPR and chest compressions can be more effectively performed.

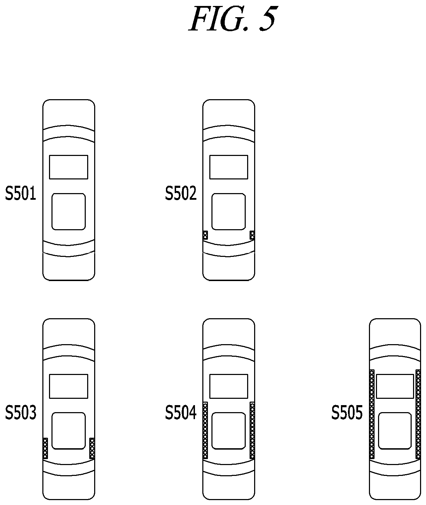

An operation of the light source operating unit 1013 will be described in detail with reference to **FIG. 5. FIG. 5** is a diagram illustrating an operation of a light source operating unit according to an exemplary embodiment of the present disclosure. Referring to **FIG. 5**, S501 to S505 show light sources operated by the light source operating unit 1013 depending on a compression depth. When the sensor unit 1011 measures a movement distance of 4 cm to 6 cm from a reference position toward the heart of a patient, the information generating unit 1011 may determine that chest compressions have been accurately performed and the light source operating unit 1013 may operate light sources on the basis of the determination result. That is, when a position is changed by 4 cm to 6 cm in an initial state as shown in S501, it is determined that chest compressions have been accurately performed, and the light source operating unit 1013 may operate light sources as shown in S505. The light sources may be operated as stacked in red, yellow, and green. Thus, the performer can visually check that the chest compressions have been accurately performed. The light source operating unit 1013 may operate light sources even when chest compressions are performed in an accurate direction on the basis of a compression direction in addition to the compression.

Meanwhile, the light source operating unit 1013 may differentiate the number or color of light sources to be operated with a first compression depth in a first compression direction from the number or color of light sources to be operated with the first compression depth in a second compression direction. For example, if a direction of chest compressions is not accurate with an accurate compression depth, the light source operating unit 1013 may differentiate the color or number of light sources to be operated. More specifically, if the chest is compressed not in a direction toward the heart of the subject with respect to the chest as a reference position but in a direction corresponding to an angle of 30 degrees with respect to the heart, the light source operating unit 1013 may operate light sources as shown in S502 to S504.

The communication unit 1014 may transmit information to the external device 20. While the user performs chest compressions, the communication unit 1014 may transmit a current position of the user to the external device 20. The external device 20 may be configured as a mobile device which is communication-connected with at least two chest compression assisting apparatuses 10. For example, when there is an emergency patient, the communication unit 1014 may transmit information about a current position to an external server or the external device 20, and according to another exemplary embodiment, when the number of chest compressions measured by the sensor unit 1011 is 30 or more, the communication unit 1014 may transmit a current position to the external server or the external device 20 in order for an emergency rescue team to receive position information of the emergency patient. In this case, the communication unit 1014 may be provided to directly communicate using a mobile communication network, Wi-Fi, or the like, or may be provided as a Bluetooth device to perform local area communication with the external device 20 carried by the performer who is performing chest compressions and thus to communicate with the external device 20 and a server. In this case, the communication unit 1014 may be used as installed as a separate application in a mobile device.

According to another exemplary embodiment, while the user performs chest compressions, the communication unit 1014 may transmit information relevant to the chest compressions to the external device 20. For example, if there is an emergency patient, when the number of chest compressions measured by the sensor unit 1011 is 30 or more, the communication unit 1014 transmits information relevant to the chest compressions to the external device 20 and the external device 20 transmits a current position measured by a GPS or the like installed in the external device 20 to an external server or a sperate device in order for an emergency rescue team to receive position information of the emergency patient.

The communication unit 1014 may be designed as a Bluetooth-based wireless communication circuit for data synchronization between the chest compression assisting apparatus 10 and the external device 20 such as a smart device application. A virtual network may be constructed using dummy files to construct a Bluetooth-based wireless network, or a network which minimizes power consumption using a Bluetooth low power profile and through which multiple chest compression assisting apparatuses 10 are connected to the external device 20 may be constructed.

More specifically, the communication unit 1014 may be connected to the external device 20 that performs monitoring and analysis of parameters relevant to CPR or chest compressions measured by the chest compression assisting apparatus 10. In this case, the external device 20 may perform monitoring and analysis through a smart device application installed in the external device 20. The communication unit 1014 may transmit information about 5 evaluation items for CPR qualification to the external device 20, and the external device 20 may monitor the information. The 5 evaluation items for CPR qualification may include, for example, a compression rate, a compression level, accurate relaxation after compression, an accurate compression axis, a frequency and a duration of compression pauses, etc.

The external device 20 according to an exemplary embodiment of the present disclosure may function as a database for storing medical records therein, may store a CPR time for use in preparing a report after CPR and use accumulated database as CPR feedback and CPR-relevant research materials. Further, the external device 20 may construct or test a virtual sensor network using a dummy file. The external device 20 may construct or test a virtual sensor network using an initial dummy file to allow a mobile application to access the database and transmit and receive data.

The switch 1015 may receive a user input to start providing information relevant to chest compressions. For example, the switch 1015 may enable the user to operate the chest compression assisting apparatus 10 and may be configured as a button. However, the switch may be provided as a touch screen to perform input and output at the same time, but is not limited thereto.

If an input is made to operate the chest compression assisting apparatus 10 through the switch 1015, the information generating unit 1012 may set a position of the sensor unit 1011 as a reference position and generate information on the basis of a measurement value measured by the sensor unit 1011. In other words, a position where the chest compression assisting apparatus 10 is applied with power through the switch can be a reference position.

If an operation of a predetermined type is input through the switch 1015, the first threshold range and the second threshold range may be changed on the basis of one of age or gender of a subject of chest compressions. For example, if the switch 1015 is quickly pressed twice, a mode may be changed, and there may be a mode having various threshold ranges depending on age or gender of a patient.

The sound output unit 1016 may output sound to guide the user about chest compressions while the user performs the chest compressions. For example, the sound output unit 1016 may sound an alarm to notify of a recommended cycle of CPR during CPR, and may be designed in the form of a buzzer to provide a regular alarm and thus notify the user of a recommended compression cycle during CPR.

According to another exemplary embodiment, the sound output unit 1016 may be provided as a speaker and may output sound 30 times at a rate of 100 to 110 per minute. The user may perform chest compressions to the sound output by the sound output unit 1016. The sound output unit 1016 may output sound having a predetermined rhythm to enable the user to perform chest compressions 30 times during CPR. Further, the sound output unit 1016 may output sound to enable the user to perform artificial respiration, and the sound for artificial respiration may be continuously output for 1 second and then silence may be output for 2 seconds, which may be repeated twice. The sound for artificial respiration may be output after the chest compressions are ended, and the sound for chest compressions and the sound for artificial respiration may be different from each other.

Although not illustrated in the drawings, a GPS (not illustrated), an LED counter (not illustrated), and an optical sensor (not illustrated) may be further included in the chest compression assisting apparatus 10 according to various exemplary embodiments of the present disclosure.

The GPS can find a current position of the user, and two or more GPSs may be provided for the accuracy in measuring a position. It is possible to obtain more accurate information by using two or more GPSs adjacent to each other and offsetting a common position of the GPSs. Then, it is possible to obtain corrected position information by comparing the information with position information received from another GPS.

The LED counter can display the number of chest compressions. More specifically, when the user performs chest compressions accurately, information may be output through the LED counter, and, thus, the user can obtain information about the number of chest compressions. The LED counter may also display time.

The optical sensor is a sensor that enables a wearer of the chest compression assisting apparatus 10 to monitor him/herself. The optical sensor monitors whether or not there is something wrong with the wearer to generate information. That is, the optical sensor senses the user's pulses and if a pulse rate is measured to be higher or lower than a predetermined value, such information is transmitted to the external device 20 through the communication unit 1014 or notified to surrounding people through the sound output unit 1016, and, thus, the optical sensor enables the wearer to ask for help in case of emergency. For example, the optical sensor may be provided as an infrared sensor to monitor the user's pulses, but is not limited thereto.

According to various exemplary embodiments of the present disclosure, the external device 20 may serve as another chest compression assisting apparatus. The other chest compression assisting apparatus may be worn on a part of a second user's body, and the other chest compression assisting apparatus may operate using information generated by the chest compression assisting apparatus 10. For example, if there are two or more performers for performing CPR, information relevant to CPR generated by the chest compression assisting apparatus 10 of a first user may be shared by a chest compression assisting apparatus of a second user, and, thus, CPR can be performed efficiently.

The other chest compression assisting apparatus may be worn on another part of the user's body, and in this case, the communication unit 1014 may receive external information from the other chest compression assisting apparatus and the information generating unit 1012 may generate information relevant to chest compressions or CPR considering the external information. In this case, the other part of the user's body may be the left wrist or right wrist of the user, but is not limited thereto.

In other words, the chest compression assisting apparatus 10 may be worn on body parts of two or more users and may operate by sharing information thereof, or may be worn two or more body parts of a single user and may operate by sharing information thereof.

Further, according to an exemplary embodiment of the present disclosure, the chest compression assisting apparatus 10 may store the external information received from the external device 20. For example, if the external device 20 is a CPR pad that provides a guide about how to perform CPR, the user may perform chest compressions using the CPR pad attached to the subject and the communication unit 1014 may receive external information from the CPR pad and the information generating unit 1012 may generate information relevant to the chest compressions and CPR considering the external information. The chest compression assisting apparatus 10 may store the received external information. The external device 20 may be an Automated External Defibrillator (AED), but is not limited thereto.

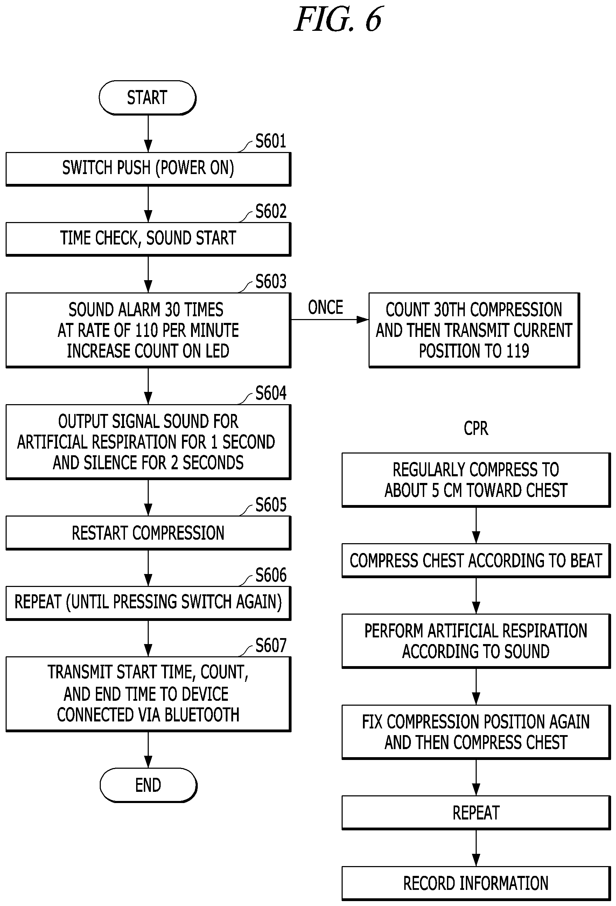

**FIG. 6** is a flowchart illustrating a process of chest compressions according to an exemplary embodiment of the present disclosure. Referring to **FIG. 6**, in a process S601, a chest compression performer may rest the palm heels of both folded hands on the center of the chest of an emergency patient to perform CPR and press the switch 1015 of the chest compression assisting apparatus 10. In this case, a mode can be changed for adult or child depending to age group of the patient by quickly pressing the switch 1015 twice.

In a process S602, audio guidance about CPR may be output, and the performer may regularly compress the chest of the subject to a depth of about 5 cm in a direction toward the heart.

In a process S603, the chest compression assisting apparatus 10 may output sound to provide a guide about chest compressions 30 times at a rate of 110 per minute and the user may regularly compress the chest to a depth of about 5 cm to the beat of the sound. While the chest compressions are performed 30 times, the chest compression assisting apparatus 10 may transmit a current position to a smart phone connected thereto through a network at the time of a 15th chest compression 15 and send a request for rescue to a 119 rescue center. Meanwhile, while performing the chest compressions, the performer may check the accuracy of the chest compressions by monitoring light sources operated in the chest compression assisting apparatus 10. If the chest compressions are accurately performed to a depth of 4 cm to 6 cm from the chest of the subject toward the heart along a Z-axis, multiple light sources may rise to be turned on in green. The light sources may be operated in red, which means that the chest compressions are not accurately performed. Meanwhile, while the chest compression assisting apparatus 10 regularly sounds an alarm 30 times, the chest compression assisting apparatus 10 may count the number of times of performing accurate chest compressions and store time and the number of times thereof. The chest compression assisting apparatus 10 may be combined in the form of a rotatory structure in which the main unit 101 is rotated by the rotating unit to enable the performer to easily check a color of the light sources.

In a process S604, after the audio guidance is provided 30 times, a sound for artificial respiration may be continuously output for 1 second and then silence may be output for 2 seconds. The sound for artificial respiration may be output twice. In this case, even if chest compressions are performed without artificial respiration, the number of the compressions may be recorded. The performer may perform artificial respiration according to the sound for artificial respiration. In this case, if a depth or cycle of chest compressions is out of a normal range, a feedback message may be output by sound or display.

In a process S605, after the sound for artificial respiration is ended, the sound may be changed to a sound for chest compressions and the performer may restart chest compressions according to the sound. In a process S606, the chest compression assisting apparatus 10 may repeat the operations of the process S603 to the process S605 until the operations are ended by the switch 1015, and the performer may perform CPR on the basis of the audio guidance. In a process S607, if the CPR or the chest compressions are ended, the switch 1015 of the chest compression assisting apparatus 10 may be operated to stop the operation of the chest compression assisting apparatus 10 and start time, end time, the number of compressions, and record graph may be transmitted to the devices connected through the network. The user may make a record on a medical chart on the basis of the transmitted information.

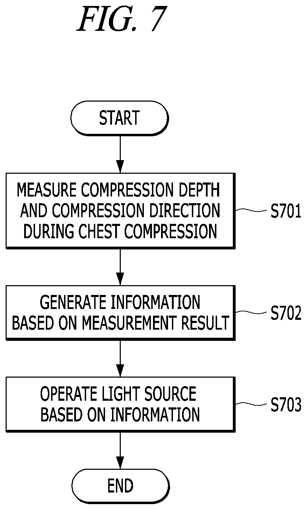

**FIG. 7** is a flowchart illustrating a method for assisting chest compressions using an apparatus according to an exemplary embodiment of the present disclosure. The method for assisting chest compressions as illustrated in **FIG. 7** can be explained by the description about the operations performed in the respective units of the chest compression assisting apparatus 10 with reference to **FIG. 1** to **FIG. 6****.** Therefore, although not described below, a detailed explanation of parts which are included in or can be inferred from the description about the operations of the chest compression assisting apparatus 10 with reference to **FIG. 1** to **FIG. 6** will be omitted.

Referring to **FIG. 7**, the chest compression assisting apparatus 10 may measure a compression depth and a compression direction during chest compressions in a process S701, generate information on the basis of a result of the measurement in a process S702, and operate a light source on the basis of the generated information in a process S703.

According to another exemplary embodiment, the chest compression assisting apparatus 10 may apply power to the chest compression assisting apparatus 10 through the switch 1015. In this case, the chest compression assisting apparatus 10 may operate to set a current position of the chest compression assisting apparatus 10 as a reference position, and then, the light source may be turned on depending on a change in position of the chest compression assisting apparatus 10.

When a position is adjusted in the chest compression assisting apparatus 10, a body of the main unit 101 may be rotated using the rotating part. This is to rotate the main unit 10 to an angle at which the user can see the main unit 10 more easily. When chest compressions are performed using the chest compression assisting apparatus 10, the sensor unit 1011 may sense a change in position with respect to the current position, and if there is a change in position corresponding to a predetermined change in position, it may be determined that the chest compressions have been accurately performed. Then, such a result of the determination maty be visually shown by operating the light source.

When chest compressions are performed using the chest compression assisting apparatus 10, the sensor unit 1011 may sense a change in position with respect to the current position, and if there is a change in position corresponding to a predetermined change in position, it may be determined that the chest compressions have been accurately performed. Then, such a result of the determination maty be visually shown by operating the light source.

More specifically, chest compressions may be performed at a rate of 100 to 110 per minute to the sound output 30 times, and a chest compression performer may perform the chest compressions to the output sound, and, thus, it is possible to provide a guide about CPR and also possible for the performer to more effectively perform CPR. After the chest compressions are performed, the chest compression assisting apparatus 10 may output a sound for artificial respiration to enable the performer to perform artificial respiration. Accordingly, after the chest compressions, the chest compression performer can perform artificial respiration to the sound output by the chest compression assisting apparatus 10 and thus perform CPR. The sound for artificial respiration may be continuously output for 1 second and then silence may be output for 2 seconds, which may be repeated twice.

The chest compression assisting apparatus 10 may transmit a current position to the external device. This is to inform the outside of a position of an emergency patient and thus quickly take actions after CPR. The chest compression assisting apparatus 10 may be provided to transmit information to an external server or device using a mobile device carried by the CPR performer through local area communication such as Bluetooth, and in this case, a separate application may be installed in the mobile device, and, thus, the mobile device can perform local area communication with the chest compression assisting apparatus 10.

The above description of the present disclosure is provided for the purpose of illustration, and it would be understood by those skilled in the art that various changes and modifications may be made without changing technical conception and essential features of the present disclosure. Thus, it is clear that the above-described embodiments are illustrative in all aspects and do not limit the present disclosure. For example, each component described to be of a single type can be implemented in a distributed manner. Likewise, components described to be distributed can be implemented in a combined manner.

The scope of the present disclosure is defined by the following claims rather than by the detailed description of the embodiment. It shall be understood that all modifications and embodiments conceived from the meaning and scope of the claims and their equivalents are included in the scope of the present disclosure.

### [EXPLANATION OF REFERENCE NUMERALS]

10: Chest compression assisting apparatus
101: Main unit
1011: Sensor unit
1012: Information generating unit
1013: Light source operating unit
1014: Communication unit

## Claims

1. An apparatus for assisting chest compressions, comprising:
a main unit that generates information relevant to the chest compressions; and
a connecting part which is extended from the main unit and of which both ends are connected to each other in order for the apparatus to be wearable on a part of a user's body,
wherein the main unit includes:
a sensor unit configured to measure a compression depth during the chest compressions and measure a compression direction during the chest compressions,
an information generating unit configured to generate information on the basis of a result of the measurement; and
a light source operating unit configured to operate a light source on the basis of the information.

2. The apparatus for assisting chest compressions of Claim 1,
wherein the light source operating unit operates multiple light sources having different colors, and
if the compression depth is within a first threshold range, the light source operating unit operates a first color light source, and if the compression depth is within a second threshold range, the light source operating unit operates both the first color light source and a second color light source.

3. The apparatus for assisting chest compressions of Claim 1,
wherein the light source operating unit turns on some of the multiple light sources depending on the compression depth, and
the number of some light sources varies depending on the compression depth.

4. The apparatus for assisting chest compressions of Claim 1,
wherein the light source operating unit differentiates the number or color of light sources to be operated with a first compression depth in a first compression direction from the number or color of light sources to be operated with the first compression depth in a second compression direction.

5. The apparatus for assisting chest compressions of Claim 2,
wherein the first threshold range and the second threshold range are determined on the basis of at least one of age or gender of a subject of the chest compressions.

6. The apparatus for assisting chest compressions of Claim 1,
wherein the sensor unit measures at least one of the compression depth and the compression direction using an acceleration sensor.

7. The apparatus for assisting chest compressions of Claim 1, further comprising:
a communication unit that transmits the information to an external device.

8. The apparatus for assisting chest compressions of Claim 1,
wherein the external device is another apparatus for assisting chest compressions.

9. The apparatus for assisting chest compressions of Claim 8,
wherein the other apparatus for assisting chest compressions is worn on a part of a second user's body, and
the other apparatus for assisting chest compressions operates using the information.

10. The apparatus for assisting chest compressions of Claim 8,
wherein the other apparatus for assisting chest compressions is worn on another part of the user's body,
the communication unit receives external information from the other apparatus for assisting chest compressions, and
the information generating unit generates the information considering the external information.

11. The apparatus for assisting chest compressions of Claim 8,
wherein the external device is a mobile device which is communication-connected with at least two apparatuses for assisting chest compressions.

12. The apparatus for assisting chest compressions of Claim 1, further comprising:
a detachable part which is formed between the main unit and the connecting part and fixes the main unit to be detached or attached.

13. The apparatus for assisting chest compressions of Claim 1,
wherein the information is accuracy information indicating whether or not the chest compressions are accurately performed.

14. The apparatus for assisting chest compressions of Claim 1,
wherein the sensor unit measures at least one of the compression depth and the compression angle on the basis of an offset and a signal change cycle depending on a change in acceleration of the main unit.

15. The apparatus for assisting chest compressions of Claim 7,
wherein the communication unit transmits a current position of the user to the external device while the user performs the chest compressions.

16. The apparatus for assisting chest compressions of Claim 1,
wherein the main unit further includes:
a switch configured to receive a user input to start providing the information relevant to the chest compressions; and
a sound output unit configured to output sound to guide the user about the chest compressions while the user performs the chest compressions.

17. The apparatus for assisting chest compressions of Claim 16,
wherein if an operation of a predetermined type is input through the switch, the first threshold range and the second threshold range are changed on the basis of at least one of age or gender of a subject of the chest compressions.

18. The apparatus for assisting chest compressions of Claim 1,
wherein the sensor unit is a 6-axis acceleration sensor, and
the sensor unit measures a movement distance from the chest in a direction toward a reference axis as the compression depth.

19. A method for assisting chest compressions using an apparatus, comprising:
generating information relevant to the chest compressions by a main unit; and
making a user wear the apparatus on a part of his/her body by extending a connecting part from the main unit and connecting both ends of the connecting part to each other,
wherein the generating of information relevant to the chest compressions includes:
measuring a compression depth during the chest compressions and a compression direction during the chest compressions,
generating the information on the basis of a result of the measurement; and
operating a light source on the basis of the information.
